# EUROPEAN PATENT APPLICATION

(11) **EP 3 042 947 A1**
(43) Date of publication of application: **13.07.2016**
(21) Application number: 14839036.2
(22) Date of filing: 19.02.2014
(51) Int. Cl.: C12M 1/107, C12M 1/42, C05F 3/06, C05F 3/00, C05F 9/00

(54) **METHOD OF PREPARING RAW MATERIAL FOR ANAEROBIC DIGESTION OF ORGANIC WASTE AND INSTALLATION FOR IMPLEMENTING SAME**

(30) Priority: 02.09.2013 RU 2013140555
(71) Applicant: "BIOENERGY" LIMITED LIABILITY COMPANY, Ekaterinburg 620016 (RU)
(72) Inventor: SMOTRITCKII, Aleksandr Andreevich, g. Ekaterinburg 620102 (RU); SMOTRITSKIY, Andrej Vladimirovich, g. Ekaterinburg 620102 (RU); SMOTRITSKAY, Tat`yana Andreevna, g. Ekaterinburg 620102 (RU)
(74) Representative: Chimini, Francesco
(86) International application number: PCT/RU2014/000103
(87) International publication number: WO 2015/030624

(57) **Abstract**

The group of inventions refers to the sphere of agriculture and bioenergetics, it can be used in the process of substrate pretreatment for anaerobic digestion of different types of organic waste into eco-friendly and useful substances in a form of fertilizers and biogas with the purpose of biological waste treatment and local stabilization of the ecological situation in the places of its concentration (animal and poultry manure, agricultural and agro-industrial wastes, waste food etc.). The group of inventions includes the substrate pretreatment method for anaerobic digestion of organic wastes and an installation for its realization. The subject matter of this group is characterized in that simultaneously with heating they make degassing of the mixture by means of vacuuming and further treatment with the ultrasonic hydrodynamic radiator energy impact on the mixture flow. Technical result of the group of inventions consists in the increase of substrate quality by means of fine milling, disinfection and homogenization of the mixture - which results in the speed increase of the digestion by 1,5 - 2,0 times and that of the product yield by 2,0 - 2,5 times.

## Description

The group of inventions refers to the sphere of agriculture and bioenergetics, it can be used in the process of substrate pretreatment for anaerobic digestion of different types of organic waste into eco-friendly and useful substances in a form of fertilizers and biogas with the purpose of biological waste treatment and local stabilization of the ecological situation in the places of wastes concentration (animal and poultry manure, agricultural and agro-industrial wastes, waste food etc.).

Perspective development of biotechnology for organic wastes disposal includes intensification of bioprocesses due to the increase of biological agents' potential and modernization of equipment. Important stage of the biotechnological production of useful products from the organic waste comprises thorough pretreatment of the substrate in order to create a nutrient medium for microorganisms' cultivation and get a high quality mix added to the main installation - bioreactor (fermenter).

There are many methods of organic wastes digestion and many installations with bioreactors, for example US 6663777 A1, 18.09.2003. FR 2614888, 1988. RU 2084515 C1, 20.07.1997. RU 2102468 C1, 20.01.1998. RU 2258686, 20.08.2004. RU 2315721 C1, 27.01.2006. RU 2370457 C1, 20.10.2009. RU 2399184 C1, 20.09.2010. RU 2404240 C2, 20.11.2010. RU 88665 U1, 20.11.2009. RU 110588 U1, 27.11.2011. RU 125995 U1, 20.03.2013.

All known designs and methods with a unified digestion technology, i.e. creation of conditions for microbiological digestion of the biomass with methane-producing anaerobic bacteria in one or several stages, implement substrate pretreatment from simple mixing to mixture fine milling before feeding to the bioreactor.

Normally when preparing the substrate it is preliminary grinded and then mixed in a tank or firstly mixed with liquid and/or additives and then grinded to the condition applicable for further digestion.

There is a method of substrate pretreatment implementing milling and mixing of the biomass with a mechanical bladed device in a tank connected via perforated plate to the acid digestion chamber of the bioreactor (RU 2084515, 20.07.1997).

In order to intensify digestion process when preparing the substrate following the patent RU 2370457, 20.10.2009 the substrate is milled and mixed with further addition of liquid collected after the wet organic fertilizer drained out of the anaerobic bioreactor.

There are known methods of organic waste treatment where mixing, milling and homogenization of organic waste is done via the electrohydraulic charges' impact on the mixture. These charges are cyclically moving along the length of the installation (RU 2135437, 27.08.1999), and methods where organic fertilizer is produced by means of organic part dispersion, hydro-percussion impact on the mixture in the process of closed loop circulation (RU 2258686, 20.08.2005) or by means of ultrasonic field vibration spectrum impact during the aerobic treatment with further magnet treatment in the process of anaerobic digestion (RU 2207328, 27.06.2003).

Known methods of substrate pretreatment when the mixture is milled mechanically removing all big and long pieces or via dispersion, when the mass is more homogenous for the bioreactor, do not provide getting the substrate of the quality acceptable for intensive microbiologic digestion.

Anaerobic digestion of unduly prepared biomass under right conditions takes much more time (2-3 times more), and biogas output is 50 - 200% less than in case of similar amount of duly prepared biomass digestion.

Substrate pretreatment method for anaerobic digestion EP 1636869, 22.05.2006 is taken as a prototype.

Prototype method consists in the fact that organic substrate is charged, mixed with water and subjected to ultrasonic radiation while the mix is passing through the channel surrounded by ultrasonic radiators. Then the substrate is fed to the anaerobic reactor.

The substrate prepared following the prototype method increases the speed of hydrolysis and the release of watersoluble substances from waste making these substances more exposed to the microorganisms. However efficiency of this method is not high as consequently installed ultrasonic radiators' operation takes much time and energy in order to achieve the desired effect. Therewith the quality of the substrate is not high enough.

Quality parameters of the substrate include not only temperature but also the degree of biomass organic substances exposure to the microorganisms' treatment. Biomass in its original state almost always consists of cell groups separated by membranes, coats, protective layers, so decomposition of the biomass goes slowly. Some cell groups remain undecomposed with its biomass digested.

Substrate quality improvement allows to increase efficiency of the bioreactor - the most energy-consuming and expensive element of the biogas complex.

The group of inventions united by one goal follows the idea to increase efficiency of the substrate pretreatment providing increase of organic waste anaerobic digestion speed and making it more intense.

Technical result of inventions group is the increase of substrate quality by means of fine milling, disinfection and homogenization of the mixture - which results in the digestion speed increase and that of the product yield while decreasing material costs.

Technical result is achieved due to the fact that the method of substrate pretreatment for anaerobic digestion of organic wastes includes substrate feeding, dosed mixing with liquid and heating up to the set temperature of the process, mixture radiation treatment and substrate charging to the bioreactor. Unlike the prototype organic waste anaerobic digestion substrate pretreatment method includes mixture heating process going simultaneously with degassing by means of vacuuming and further mixture flow treatment with the energy of the ultrasonic hydrodynamic radiator.

It is feasible to perform all manipulations in one airtight tank, so ultrasonic hydrodynamic treatment of the mixture is done while it circulates in the closed loop of the airtight tank. Therewith non-oxygen gas is being supplied to the chamber of the ultrasonic hydrodynamic bioreactor. This gas can be supplied directly from the bioreactor, and liquid drained fraction from the bioreactor is used as a liquid for mixing the substrate.

Proposed method is realized with the help of the substrate pretreatment installation developed, tested and described below. Realization of the method is also possible on the substrate pretreatment flow line by means of charging through consequently installed equipment.

Mechanical milling and mixing devices are traditionally used in the process of substrate pretreatment for organic wastes digestion with further addition of liquid (if necessary) and/or other components with or without heating.

Substrate pretreatment without milling (utility model RU 88665, 20.11.2009) does not provide homogeneous granulometric composition of the bioreactor substrate, and it slows down the process of biodigestion.

Above mentioned flaw is eliminated in the substrate pretreatment block of the process line under the utility model patent RU 125995, 20.03.2013 containing storage hopper and a grinder with the possibility to charge large sized substrate in the grinder, return of the grinded substrate to the feeding hopper and charging grinded substrate from the feeding hopper to the loading transporter - mixer. This pretreatment of the substrate requires a lot of equipment and also does not provide optimal quality of the substrate.

Mechanical rotary shredders are widely used as grinding and mixing tools for the biomass, for instance bladed units with driving shaft and blades installed in the tanks interconnected via perforated wall and equipped with a chamber of acid digestion of the reactor (RU 2084515, 20.07.1997).

In order to increase digestion process efficiency in the system of substrate pretreatment under the patent RU 2370457, 20.10.2009 there are additional feeding mechanisms for liquid supply to the tank with the grinder. This liquid comes from the wet organic fertilizer drained from the anaerobic bioreactor.

There is also an installation for organic waste processing into fertilizers where the mixing, milling and homogenization tools are made in a form of electrohydraulic chamber with a row of paired electrodes placed inside (RU 2135437, 27.08.1999), biogas unit under the patent RU 2102468, 20.01.1998, where substrate pretreatment is realized with the use of a decomposer located between the manure collector and the bioreactor, and installations for organic fertilizers' production by means of mixture dispersion under the patents RU 2258686, 20.08.2005 and RU 2207328, 27.06.2003, in the first one there is a rotary hydro-percussion device for hydro-impacting on the mixture while it circulates in the closed loop, in the second there is an ultrasonic vibration generator for the ultrasonic impact on the mixture in the process of anaerobic treatment.

Known substrate pretreatment units with mechanical grinders and decomposers removing large and long fragments preventing mixture pumping to the bioreactor and methods realized there are ineffective and do not provide due quality substrate making the design of bioreactors for further anaerobic treatment of organic wastes more difficult.

Biological waste digestion unit EP 1636869, 22.05.2008 substrate pretreatment system is taken as a prototype for the substrate pretreatment installation for anaerobic digestion of wastes.

Pretreatment system as per prototype includes substrate and water mixing device and a number of ultrasonic radiators for ultrasonic treatment located round the bioreactor substrate access channel. Radiator device corresponds to that one described in WO 0335579, 22.06.2000, consisting of a tank and many ultrasonic probes attached to the outer wall of the tank. Sixty modules of 50 kW piezoelectric transducers each resonating at 20 kHz are tightly packed in a grid and form five rings around the tank, 12 modules per each.

Quite complex design prototype substrate pretreatment installation and the realized method are ineffective and do not provide high quality substrate, they take much time for processing and high material cost.

The above mentioned technical result of the invention group is also achieved due to the fact that the organic waste anaerobic digestion substrate pretreatment installation includes devices for dozing substrate and water, heating, radiation treatment and substrate dozing into the bioreactor. As opposed to the prototype organic waste anaerobic digestion substrate pretreatment installation is equipped with an airtight tank furnished with a vacuum pump, and mixture radiation treatment device is represented by an ultrasonic hydro-dynamic radiator and its chamber is connected to the tank making a closed loop.

Furthermore the ultrasonic hydrodynamic radiator chamber is equipped with non-oxygen gas supply device which can be connected to the bioreactor, and the liquid supply device for substrate mixing - to the bioreactor drainage system.

Claimed group of inventions is clarified with a schematic drawing of the substrate pretreatment installation for organic waste anaerobic digestion used for the realization of the claimed method.

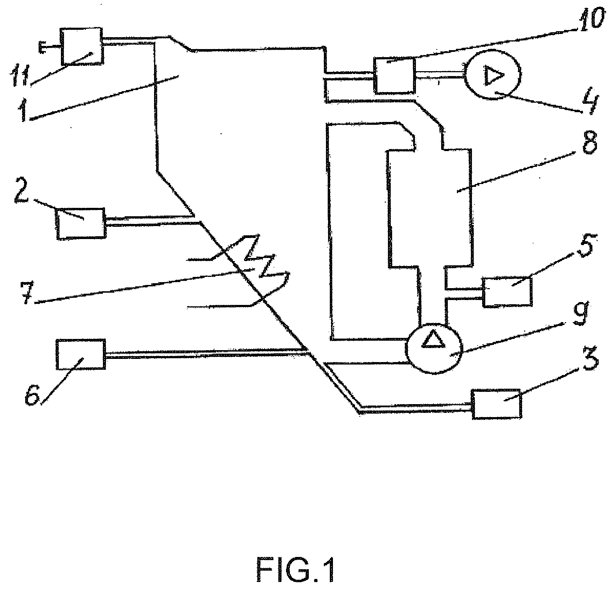

The unit includes an airtight tank 1 connected to the organic substrate and liquid dosimeters 2 and 3 accordingly, with vacuum pump 4, dosimeter 5 for gas supply from the bioreactor and dosimeter 6 of the substrate supply to the bioreactor (not shown on the drawing). Tank 1 is equipped with a heater 7 and an ultrasonic hydrodynamic radiator 8 of a laminar or rod type, its chamber is connected to the tank 1 via purge pump 9 forming a closed loop of the mixture circulation.

Substrate pretreatment method is realized in the operation of the installation in a following way.

Substrate from the storage hopper and elevated temperature liquid from the wet organic fertilizer drained from the anaerobic reactor are simultaneously supplied to the airtight tank 1 through the dosimeters 2 and 3 under pressure. Upon completion of charging the temperature of the mixture is measured, then heater 7 is turned on in order to get the necessary temperature of the bioreactor 1 stage process, which is usually 37°C. Vacuum pump 4 turns on and simultaneously with the mixture heating the vacuuming process starts in the same airtight tank lasting for 15 - 20 minutes. Vacuuming helps to evacuate gas vapors and to remove the major part of oxygen saluted in the substrate being poisonous for the microorganisms participating in the biotechnological process. Then pump 4 is switched off, line is closed by valve 10, pump 9 switches on, ultrasonic hydrodynamic radiator 8 starts and gas supply devices are connected via dosimeter 5 from the bioreactor. Mixture heating and circulation in a closed loop via the ultrasonic hydrodynamic radiator 8 go simultaneously. Surplus of gas is blown off via valve 11.

As a result of repeated mixture circulation in the ultrasonic hydrodynamic radiator 8 the vortices of fluid shed by the surface of mechanical resonators and the oscillations of these resonators excite harmonic acoustic oscillations in the fluid in a form of periodic pressure impulses. Periodic impulses simulate oscillations providing feedback and continuous acoustic oscillations. As a result the amplitude of excited harmonic acoustic oscillations increases under the conditions of resonance with oscillations of the radiator 8 mechanical resonators' oscillations. Increased treatment cavity zone is formed and it influences the cell structure of the biomass by breaking the cell walls and providing intensive milling of the mixture. Under the influence of acoustic and ultrasonic waves excess pressure and tensile stresses appear in the fluid, hence oxidation-reduction processes start at the border of liquid and solid phases, so stable emulsion and suspension with high dispersion particles are acquired. Additional supply of non-oxygen gas to the cavity zone via dosimeter 5 facilitates the process of developed cavity flow mode creation, optimizes the process and generates many tiny carbon dioxide bubbles. These bubbles go to the bioreactor and serve as food for the methanogens, i.e. they promote increase of methane content in the biogas. Processing lasts for 10 - 30 minutes and finishes when the mixture is heated to the needed temperature. After the treatment the substrate is pumped to the bioreactor through dosimeter 6. Then the pretreatment process is repeated.

Installation operation is fully automatized.

Thus within the airtight tank the mixture is simultaneously heated, degassed and thoroughly treated, it significantly increases efficiency of the process of high quality substrate pretreatment of any type.

Installation equipped with the tank of 1,5 m³ and a purge pump with capacity of 20-50 m³/hour, provides pretreatment of 50 m³ of substrate per day.

Experiments prove that the duration of chicken manure digestion by the proposed method in the claimed substrate pretreatment installation decreased from 14 to 9 days, and the amount of received biogas increased 2,2 times. Moreover, as a result of oxygen removal from the mixed components the reaction started faster and carbon dioxide contents in the biogas decreased.

## Claims

1. Substrate pretreatment method for anaerobic digestion of organic wastes including substrate charging, dozed mixing with liquid and heating up to the needed process temperature, mixture treatment with radiation and substrate charge to the bioreactor, **characterized in that** simultaneously with heating they make degassing of the mixture by means of vacuuming and further treatment with the ultrasonic hydrodynamic radiator energy impact on the mixture flow.

2. The method of clause 1 **characterized in that** the ultrasonic hydrodynamic mixture treatment is done while the mixture circulates in the closed loop of an airtight tank.

3. The method of clause 1 **characterized in that** gas, containing no oxygen, is added to the chamber of the ultrasonic hydrodynamic radiator.

4. The method of clause 1 **characterized in that** gas supply is done directly from the bioreactor.

5. The method of clause 1 **characterized in that** the liquid fraction drained from the bioreactor is used as liquid for substrate mixing.

6. Substrate pretreatment installation for organic waste anaerobic digestion including devices for dozed substrate and liquid charge, heating, radiation treatment and dozed supply of the substrate to the bioreactor **characterized in that** it is equipped with an airtight tank furnished with a vacuum pump, and the mixture radiation treatment device is made in a form of an ultrasonic hydrodynamic radiator with its chamber connected to the tank forming a closed loop.

7. The installation as of clause 6 **characterized in that** the ultrasonic hydrodynamic radiator chamber is equipped with non-oxygen gas supply unit.

8. The installation as of clause 6 **characterized in that** the gas supply unit is connected to the bioreactor.

9. The installation as of clause 6 **characterized in that** the liquid supply unit is connected to the bioreactor draining system.
